# EUROPÄISCHE PATENTSCHRIFT

(11) **EP 2 384 871 B1**
(45) Veröffentlichungstag und Bekanntmachung des Hinweises auf die Patenterteilung: **07.09.2016**
(21) Anmeldenummer: 11003130.9
(22) Anmeldetag: 14.04.2011
(51) Int. Cl.: B29B 7/74, F16N 5/02, B05C 17/015

(54) **Austragsvorrichtung für Kartuschen**
Application device for cartridges
Dispositif de sortie pour cartouches

(30) Priorität: 04.05.2010 DE 102010019222
(43) Veröffentlichungstag der Anmeldung: 09.11.2011
(73) Patentinhaber: Heraeus Medical GmbH, 61273 Wehrheim (DE)
(72) Erfinder: Vogt, Sebastian, Dr., 99092 Erfurt (DE); Büchner, Hubert, Dr., 90491 Nürnberg (DE)
(74) Vertreter: Heraeus IP

(56) Entgegenhaltungen:
- EP-A1- 1 118 313
- DE-U1-202005 010 206
- US-A- 2 446 501
- US-A- 4 441 629
- US-A1- 2006 208 000

## Beschreibung

Die vorliegende Erfindung betrifft eine Austragsvorrichtung für Kartuschen umfassend einen Abzugsgriff zum Bedienen eines Ventils der Austragsvorrichtung, einen Anschluss zum Einspeisen eines unter Druck stehenden Gases, ein Kartuschenanschlussstück zum Anschließen einer Kartusche und einen Grundkörper, in dem wenigstens ein Durchgang verläuft, durch den das unter Druck stehende Gas zum Kartuschenanschlussstück leitbar ist, wobei das Kartuschenanschlussstück eine Öffnung zu dem Durchgang umfasst, so dass bei Beaufschlagung eines Bodens einer im Kartuschenanschlussstück angeordneten Kartusche mit dem unter Druck stehenden Gas, der Inhalt aus der Kartusche austreibbar ist, und wobei der Durchgang durch das bedienbare Ventil schließbar und zu öffnen ist.

Gegenstand der Erfindung ist also eine Austragsvorrichtung zum Auspressen von fließfähigen

Massen aus Kartuschen. Die Austragsvorrichtung ist insbesondere für das Austragen von Knochenzementen bestimmt. Das Auspressen von Kartuschen mit Hilfe von komprimierten Gasen ist seit Jahrzehnten bekannt.

Siehe hierza als mächster Stand der Technik US 2 446 501. Die US 2,818,899 schlägt eine Dichtungspistole vor, die im Griffstück eine Gaspatrone enthält. Das komprimierte Gas der Gaspatrone drückt nach Öffnung der Patrone einen Kolben innerhalb einer Kartusche in Richtung Kartuschenkopf. Die Steuerung des Flusses der pastösen Masse erfolgt durch einen zentralen durch die Kartusche gehenden Stab, der die Austrittsöffnung der Kartusche verschließen kann.

In der US 3,938,709 von 1976 wird eine Austragsvorrichtung beschrieben, bei der durch Gasdruck eine Tube, die sich Innerhalb des hohlen Pistolenkörpers befindet, ausgedrückt wird. Der Gasfluss wird dabei durch ein einfaches Stiftventil mit Feder erreicht, das durch einen Handhebel betätigt werden kann. Eine Vorrichtung zum Ablassen des Gases war dabei nicht vorhanden. Das bedeutet, dass trotz der Unterbrechung der Gaszufuhr die Pistole das Material durch den bestehenden Restdruck nachdrückt.

Die EP 0 169 533 A2 von 1985 offenbart ein Einspritzgerät für viskose Mittel. Bei diesem fährt nach Unterbrechung der Druckgaszufuhr das Auspressen nicht fort, weil an der Abgabeöffnung ein Einspritzsteuerventil angeordnet ist, das den Strom des viskosen Mittels unterbricht. Interessant ist dabei, dass durch das Ventil des Abzugsgriffs einmal die Gaszufuhr und gleichzeitig der Austritt des viskosen Mittels gesteuert werden kann. Das Einspritzsteuerventil schließt, wenn keine Beaufschlagung mit komprimiertem Gas erfolgt.

In der US 4,925,061 wird ein ähnliches System beschrieben. Bei dem jedoch das Einspritzsteuerventil über eine Stange betätigt wird, die mit dem Abzugsgriff verbunden ist.

Die EP 1 118 313 A1 offenbart eine Pistole zum Auspressen von Knochenzement. Der Antrieb erfolgt hierbei ebenfalls durch eine Gaspatrone. Wesentlich ist, dass dieses sehr komplexe System eine Stange aufweist, die dazu bestimmt ist, die im Austragsrohr enthaltene Zementrestmenge auszutreiben. Diese elegante technische Lösung ist für konventionelle Polymethylmethacrylat-Knochenzemente sehr gut geeignet. Für Kartuschensysteme zur Mischung mehrerer Komponenten mit statischem Mischer ist diese Pistole jedoch nicht verwendbar. Zudem ist die Fertigung dieser Pistole sehr aufwendig.

In der US 2004/0074927 A1 wird eine Auspresspistole beschrieben, welche im Wesentlichen die gleichen Merkmale wie bei US 4,925,061 offenbart.

In den Druckschriften US 2005/0230433 A1, US 2005/0247740 A1 und US 6,935,541 B1 wird die im Grunde gleiche technische Lösung vorgeschlagen, die aus der EP 0 169 533 A2 bereits bekannt ist.

Die WO 2008/109439 A1 offenbart eine durch komprimiertes Gas betriebene Austragsvorrichtung, die ein Hydraulik-Medium verwendet, auf welche das komprimierte Gas drückt.

Es ist festzustellen, dass die bisher bekannten Austragsvorrichtungen, die mit Gaspatronen angetrieben werden und einen komplexen mechanischen Aufbau besitzen, für die Fertigung als Einmalartikel nur bedingt oder überhaupt nicht geeignet sind. Besonders die vorgeschlagenen Ventile sind sehr teuer und stellen dadurch die Verwendung der Austragsvorrichtungen als Einmalartikel in Frage. Die vorgeschlagenen technischen Lösungen lassen sich zudem nur schwer als Kunststoffspritzteile ausführen.

Der Erfindung liegt daher die Aufgabe zu Grunde, eine einfache, unkompliziert zu handhabende und kostengünstig herzustellende Austragsvorrichtung für fließfähige Massen zu entwickeln. Unter dem Begriff fließfähige Massen werden im Sinne der vorliegenden Erfindung sowohl flüssige, zähflüssige, als auch hochviskose, zähe Massen, die nur bei Druckbeaufschlagung fließen, verstanden. Das Austragen der fließfähigen Massen soll durch komprimiertes Gas erfolgen, das beispielsweise einer einsetzbaren Gaskartusche oder Druckgaspatrone entnommen wird, die sich in der Vorrichtung befindet. Diese Vorrichtung soll aus möglichst wenigen Teilen bestehen, die durch kostengünstiges Spritzgießen mit konventionellen Kunststoffen hergestellt werden können. Dafür ist es erforderlich, ein unkompliziertes, einfaches Ventilsystem zu entwickeln, das eine Kontrolle des Gasflusses innerhalb einer Verwendungszeit der Austragsvorrichtung von maximal 5 Minuten ermöglicht. Die Austragsvorrichtung soll mit wenigen Handgriffen montierbar sein und unkompliziert in großen Stückzahlen hergestellt werden können. Angestrebt wird weiterhin, dass die Austragsvorrichtung als einmal zu gebrauchender Wegwerfartikel geeignet ist. Vorteilhaft wäre es zudem, wenn der Anwender den Betriebszustand der Austragsvorrichtung an der Außenseite erkennen könnte.

Diese Aufgaben werden dadurch gelöst, dass das Ventil ein Ventilkörper ist, der drehbar im Grundkörper angeordnet ist, wobei eine Bewegung des Abzugsgriffs zu einer Drehung des Ventilkörpers im Grundkörper führt und der Durchgang im oder am Ventilkörper angeordnet ist, so dass in einer ersten Position des Ventilkörpers der Durchgang geschlossen ist und in einer zweiten Position des Ventilkörpers der Durchgang im oder am Ventilkörper das unter Druck stehende Gas zu dem Kartuschenanschlussstück leitet. Im oder am Ventilkörper ist ein zweiter Durchgang angeordnet, der in einer dritten Position des Ventilkörpers das Kartuschenanschlussstück mit einem ersten Ablass verbindet, so dass ein Überdruck am Kartuschenanschlusstück in der dritten Position des Ventilkörpers durch den ersten Ablass entweichen kann.

Dabei kann vorgesehen sein, dass zwischen zumindest einem Durchgang und dem Kartuschenanschlussstück zumindest ein vorderer Kanal im Grundkörper angeordnet ist und/oder zwischen zumindest einem Durchgang und dem Anschluss zum Einspeisen des unter Druck stehenden Gases zumindest ein hinterer Kanal angeordnet ist.

Ferner wird erfindungsgemäß vorgeschlagen, dass ein erster Ablasskanal im Grundkörper derart angeordnet ist, dass er in der dritten Position des Ventilkörpers den zweiten Durchgang mit dem ersten Ablass verbindet.

Mit der Erfindung wird zudem vorgeschlagen, dass ein dritter Durchgang im oder am Ventilkörper angeordnet ist, der einen Abzweig umfasst und der in einer vierten Position des Ventilkörpers das Kartuschenanschlussstück und das unter Druck stehende Gas mit einem zweiten Ablass verbindet, so dass ein Überdruck in der Austragsvorrichtung in der vierten Position des Ventilkörpers durch den zweiten Ablass entweichen kann.

Dabei kann vorgesehen sein, dass zwischen dem dritten Durchgang und dem Anschluss zum Einspeisen des unter Druck stehenden Gases ein zweiter hinterer Kanal im Grundkörper angeordnet ist und/oder ein zweiter Ablasskanal im Grundkörper derart angeordnet ist, dass er in der vierten Position des Ventilkörpers den Abzweig des dritten Durchgangs mit dem zweiten Ablass verbindet.

Ebenso kann es vorteilhaft sein, dass an zumindest einem der Ablässe ein Sterilfilter angeordnet ist und/oder in zumindest einem der Ablässe ein Überdruckventil angeordnet ist.

Erfindungsgemäße Austragsvorrichtungen können sich des Weiteren dadurch auszeichnen, dass der Abzugsgriff fest an der Unterseite des drehbaren Ventilkörpers angeordnet ist, so dass mit dem Abzugsgriff der Ventilkörper im Grundkörper drehbar ist.

Zudem kann es vorteilhaft sein, dass am drehbaren Ventilkörper ein Positionsanzeiger, insbesondere an der Oberseite, angeordnet ist, an dem die Position des Ventilkörpers im Grundkörper ablesbar ist.

Auch wird vorgeschlagen, dass an der Unterseite des Grundkörpers ein Griff zum Halten der Austragsvorrichtung angeordnet ist.

Ferner kann vorgesehen sein, dass der Ventilkörper ein konisch geformter, zumindest bereichsweise rotationssymmetrischer Körper ist, der in einer zur Form des Ventilkörpers passenden konischen Öffnung im Grundkörper drehbar gelagert ist

Dabei kann vorgesehen sein, dass die Außenwände des Ventilkörpers bis auf die Öffnungen der Durchgänge dicht mit den Wänden der konischen Öffnung im Grundkörper in jeder Position des Ventilkörpers abschließen, so dass die Verbindungen der Durchgänge zu zumindest einem Kanal im Grundkörper dicht sind.

Als Kanäle kommen dabei die vorderen und hinteren Kanäle im Grundkörper, sowie die Ablasskanäle im Grundkörper in Frage.

Dabei kann vorgesehen sein, dass im Grundkörper eine Ausnehmung für einen Keil vorgesehen ist, wobei ein in die Ausnehmung eingeschobener Keil den Ventilkörper in die konische Öffnung im Grundkörper presst, so dass der Ventilkörper einen gasdichten Presssitz in der konischen Öffnung erzeugt.

Auch wird vorgeschlagen, dass an der dem Kartuschenanschlussstück abgewandten Seite des Grundkörpers ein Hohlraum für eine Gaspatrone vorgesehen ist, wobei derAnschluss in dem Hohlraum angeordnet ist.

Dabei kann vorgesehen sein, dass der Anschluss einen Dom zum Öffnen einer Gaspatrone umfasst.

Auch kann vorgesehen sein, dass am Grundkörper Befestigungsmittel zum Befestigen einer Verschlusskappe mit Befestigungsmitteln angeordnet sind, wobei die Gaspatrone durch das Befestigen der Verschlusskappe zu öffnen ist

Dabei kann ferner vorgesehen sein, dass die Verschlusskappe zusätzlich ein Arretierungsmittel umfasst, das in eine Arretierungsvorrichtung im Hohlraum greift, wobei besonders bevorzugt die Arretierungsvorrichtung eine eingesetzte Gaspatrone geeigneter Größe im Hohlraum positioniert.

Ferner kann vorgesehen sein, dass die Durchgänge im Wesentlichen gerade durch das Innere des Ventilkörpers verlaufen und die Durchgänge um einen Winkel von 10° bis 80° um die Drehachse des Ventilkörpers gegeneinander versetzt sind.

Weiterhin soll die Austragsvorrichtung so beschaffen sein, dass mindestens zwei im Durchmesser unterschiedliche Kartuschen angeschlossen werden können. Dadurch wäre es möglich, pastöse Massen aus unterschiedlichen Kartuschensystemen mit nur einer Austragsvorrichtung auszupressen. Diese Eigenschaft würde wirtschaftliche Vorteile hinsichtlich der Amortisation der Austragsvorrichtung bringen.

Zur Lösung dieser Aufgabe wird mit der Erfindung zudem vorgeschlagen, dass am vorderen Rand des ersten Kartuschenanschlussstücks ein zweites Kartuschenanschlussstück mit einem größeren Querschnitt und einer zweiten Öffnung angeordnet ist, wobei das zweite Kartuschenanschlussstück zum Anschließen einer größeren Kartusche geeignet ist, und wobei die zweite Öffnung des zweiten Kartuschenanschlussstücks so groß ist, dass eine Kartusche durch die zweite Öffnung hindurch am ersten Kartuschenanschlussstück anschließbar ist.

Dabei kann vorgesehen sein, dass die Kartuschenanschlussstücke zylindrische Hohlkörper mit einer geschlossenen Seite sind, wobei die geschlossene Seite des zweiten Kartuschenanschlussstücks die zweite Öffnung umfasst und die geschlossene Seite des ersten Kartuschenanschlussstücks die erste Öffnung umfasst.

Zudem kann es vorteilhaft sein, dass auf der Innenseite der Kartuschenanschlussstücke Fixierungsvorrichtungen zum Fixieren von Kartuschen angeordnet sind.

Dabei kann vorgesehen sein, dass die Fixierungsvorrichtungen in zwei konzentrischen Kreisen an den Kartuschenanschlussstücken angeordnet sind.

Ferner wird vorgeschlagen, dass die Fixierungsvorrichtungen Gewinde, insbesondere Innengewinde, sind.

Alternativ dazu kann vorgesehen sein, dass die Fixierungsvorrichtungen Rastvorrichtungen, insbesondere in Form von in eine Richtung deformierbarer Zapfen umfassen.

Der Erfindung liegt somit die überraschende Erkenntnis zugrunde, dass die Ventilfunktion durch den erfindungsgemäßen Aufbau mit einem drehbaren Ventilkörper, der mit dem Grundkörper Durchgänge bildet, oder in dessen Inneren Durchgänge angeordnet sind, stark vereinfacht ist, um diese Funktion mit kostengünstigen Kunststoffteilen verwirklichen zu können. Das Austragen von Knochenzementen erfolgt üblicherweise im Bereich 30 Sekunden bis 5 Minuten nach dem Anmischen des Zements. Das bedeutet, das Ventil muss einen Gasstrom nur maximal 5 Minuten steuern können, die Dichtungsfunktion muss also nur wenige Minuten gewährleistet sein. Es kann angenommen werden, dass das Ventil höchstens fünfmal geöffnet und verschlossen wird. Daher Ist eine derart einfache Ventilfunktion ausreichend.

Ein Anschluss zum Einspeisen eines unter Druck stehenden Gases kann im Sinne der vorliegenden Erfindung ein hohler Dorn sein, auf den eine Kartusche aufgedrückt werden kann, die sich dabei öffnet. Ebenso kommt aber auch ein Rohrstück mit Gewinde als Anschluss in Frage, auf den eine Druckgaspatrone aufschraubbar ist. Auch ein Schlauchstück, der auf einen Anschluss einer Gaspatrone aufsteckbar ist, oder ein Stutzen, auf dem ein Schlauch von einem Kompressor als Druckgasquelle anschließbar ist, ist ein Anschluss zum Einspeisen eines unter Druck stehenden Gases im Sinne der vorliegenden Erfindung. Auch wenn die Druckgasquelle, beispielsweise als Teil des Grundkörpers, fest mit der Austragsvorrichtung verbunden ist, ist die Mündung der Druckgasquelle in einen Durchgang oder in einen Kanal Im Grundkörper der Austragsvorrichtung ein Anschluss zum Einspeisen eines unter Druck stehenden Gases im Sinne der vorliegenden Erfindung.

Die vordere Seite ist im Sinne der vorliegenden Erfindung der Teil der Austragsvorrichtung, der zum Einsetzen der Kartusche geeignet ist. Die Kartusche wird also von Vorne in das Kartuschenanschlussstück eingesetzt. Hinten ist dementsprechend der gegenüberliegende Teil der Austragsvorrichtung. Die untere Seite der Austragsvorrichtung ist die, die im Gebrauch nach unten gehalten wird.

Ein Sterilfilter für einen Auslass kann aus medizinischem Papier, gasdurchlässigen Kunststoffmembranen, gasdurchlässigen Kunststoffporenscheiben, gasdurchlässigen Metallporenscheiben, gasdurchlässigen Glasporenscheiben oder aus gasdurchlässigen Kunststoffvliesen gefertigt sein.

Es wird auch erfindungsgemäß vorgeschlagen, dass der Abzugsgriff U-förmig gestaltet ist

Auch kann vorgesehen sein, dass die Austragsvorrichtung im Wesentlichen aus medizinisch akzeptablen Kunstoffen gefertigt ist

Mit der Erfindung wird auch die Verwendung einer solchen Austragsvorrichtung vorgeschlagen, zum Auspressen von fließfähigen Massen aus Kartuschen, wobei die fließfähigen Massen in den Kartuschen gelagert sind und zum Auspressen von fließfähigen Massen, die in Beuteln gelagert sind, die sich in Kartuschen befinden.

Quellen für das unter Druck stehende Gas können Kompressoren oder Patronen sein, wobei die Patronen mit einem komprimierten Gas, beziehungsweise einem verflüssigten Gas gefüllt sein können. Beispielsweise können Kohlendioxidpatronen verwendet werden.

Im Folgenden werden Ausführungsbeispiele der Erfindung anhand von sieben schematisch dargestellten Zeichnungen erläutert. Dabei zeigt:
Figur 1: eine Aufsicht auf die Seite einer erfindungsgemäßen Austragsvorrichtung;
Figur 2: eine Aufsicht auf die Vorderseite der erfindungsgemäßen Austragsvorrichtung nach Figur 1;
Figur 3: eine Detailansicht eines Fixierungselements nach Figur 1;
Figur 4: eine Querschnittansicht einer zweiten erfindungsgemäßen Austragsvorrichtung;
Figur 5: eine Querschnittansicht eines Ventilkörpers für eine erfindungsgemäße Austragsvorrichtung nach Figur 4;
Figur 6: eine perspektivische Ansicht des Ventilkörpers nach Figur 5; und
Figur 7: eine Aufsicht auf die Oberseite einer dritten erfindungsgemäßen Austragsvorrichtung.

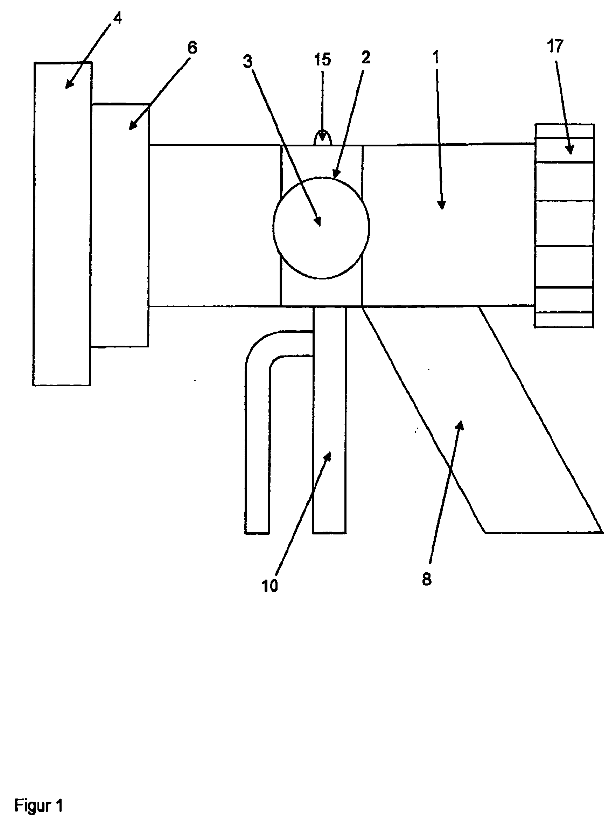

Figur 1 zeigt eine Aufsicht auf die Seite einer erfindungsgemäßen Austragsvorrichtung mit einem Grundkörper (1) mit zylindrischer Form, in dem eine seitliche Öffnung (2) in Form einer Bohrung vorgesehen ist. In der Öffnung (2) befindet sich ein in der Öffnung (2) drehbar gelagerter Ventilkörper (3) mit zylindrischer Geometrie, der mit Durchgängen im Ventilkörper (3) und im Grundkörper (1) ein Ventil bildet, so dass bei zwei unterschiedlichen Positionen des Ventilkörpers (3) zumindest zwei unterschiedliche Durchgänge durch den Ventilkörper (3) und den Grundkörper (1) geöffnet sind, während die anderen Durchgänge geschlossen sind. Die Form des Ventilkörpers (3) ist derart an die Form der Öffnung (2) angepasst, dass die Wände des Ventilkörpers (3) dicht mit der Öffnung (2) abschließen.

An der Vorderseite (in Figur 1 links) der Austragsvorrichtung ist ein erstes Kartuschenanschlussstück (4) mit einem großem Durchmesser und ein zweites Kartuschenanschlussstück (6) mit kleinerem Durchmesser angeordnet Die beiden Kartuschenanschlussstücke (4, 6) sind als zylindrische Hohlkörper ausgeformt, die auf der Vorderseite offen sind und auf der Rückseite (in Figur 1 rechts) Wände mit Öffnungen aufweisen. Die einzige Öffnung des großen Kartuschenanschlussstücks (4) ist dabei so groß, dass der Innenraum des kleinen Kartuschenanschlussstücks (6) nicht überdeckt wird. Die zumindest zwei Öffnungen in der Rückwand des kleinen Kartuschenanschlussstücks (6) führen zu den zumindest zwei Durchführungen im Grundkörper (1).

An der Unterseite der Austragsvorrichtung (in Figur 1 unten) ist ein Pistolengriff (8) angeordnet Der Ventilkörper (3) ist an der Unterseite der Austragsvorrichtung mit einem Abzugsgriff (10) und an der Oberseite mit einem Positionsanzeiger (15) verbunden, die mit dem Ventilkörper (3) relativ zum Grundkörper (1) drehbar sind. Am Grundkörper (1) ist eine Beschriftung vorgesehen (nicht gezeigt), die dem Positionsanzeiger (15) zugeordnet ist, so dass abzulesen ist, welcher der Durchgänge durch den Ventilkörper (3) gerade geöffnet ist, beziehungsweise ob das Ventil geschlossen ist. Der Abzugsgriff (10) ist aus zwei Holmen U-förmig aufgebaut, so dass ein Anwender der Austragsvorrichtung, wenn die Finger zwischen den beiden Holmen greifen, den Abzugsgriff (10) leicht sowohl zum Pistolengriff (8) hin ziehen, als auch vom Pistolengriff (8) weg drücken kann.

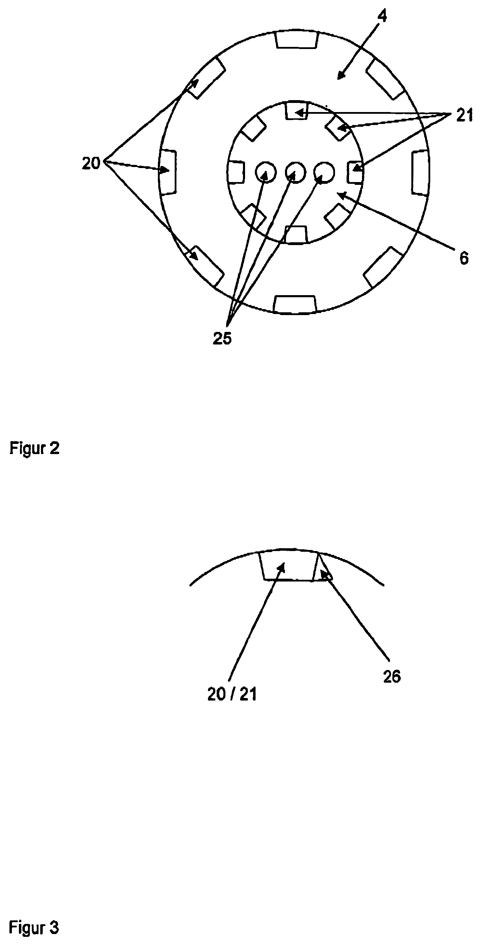

Figur 2 zeigt eine Aufsicht auf die Vorderseite der erfindungsgemäßen Austragsvorrichtung nach Figur 1. Als äußerer Ring ist das erste Kartuschenanschlussstück (4) zu erkennen, als innerer Ring das zweite Kartuschenanschlussstück (6). An den Zylinderinnenwänden der Kartuschenanschlussstücke (4, 6) sind Fixierungsvorrichtungen (20, 21) in konzentrischen Kreisen angeordnet, mit denen Kartuschen (nicht gezeigt) in den Kartuschenanschlussstücken (4, 6) fixiert werden können. Die Fixierungsvorrichtungen (20, 21) können durch Gewinde, Gewindeausschnitte oder Zapfen zum Arretieren der Kartuschen in den Kartuschenanschlussstücken (4, 6) realisiert sein.

In der rückseitigen Wand des zweiten, inneren Kartuschenanschlussstücks (6) befinden sich drei Öffnungen (25), die zu drei Durchgängen im Grundkörper (1) führen. In den Kartuschenanschlussstücken (4, 6) fixierte Kartuschen schließen dicht mit den Kartuschenanschlussstücken (4, 6) ab. Dadurch wird gewährleistet, dass ein Gasdruck, der durch eine der Öffnungen (25) auf den Boden einer fixierten Kartuschen gerichtet wird, auch auf diesen Kartuschenboden wirken kann, und nicht zwischen den Kartuschen und den Kartuschenanschlussstücken (4, 6) entweicht. Hierzu können Dichtungselemente (nicht gezeigt) in den Kartuschenanschlussstücken (4, 6) und/oder an den Kartuschen, insbesondere an den Kartuschenböden, vorgesehen sein. Es kann aber auch ausreichend sein, eine dichte Fixierungsvorrichtung (20, 21) vorzusehen, die beispielsweise durch ein Gewinde realisiert sein kann.

Figur 3 zeigt eine Fixierungsvorrichtung (20, 21) nach Figur 2, die eine Rastvorrichtung (26) umfasst, mit der eine Kartusche einem Kartuschenanschlussstück (4, 6) einrastbar ist An den Fixierungsvorrichtungen (20, 21) zur Fixierung der Kartusche können also Rastvorrichtungen (26) in Form von in einer Richtung deformierbarer oder elastisch verformbarer Zapfen angebracht sein.

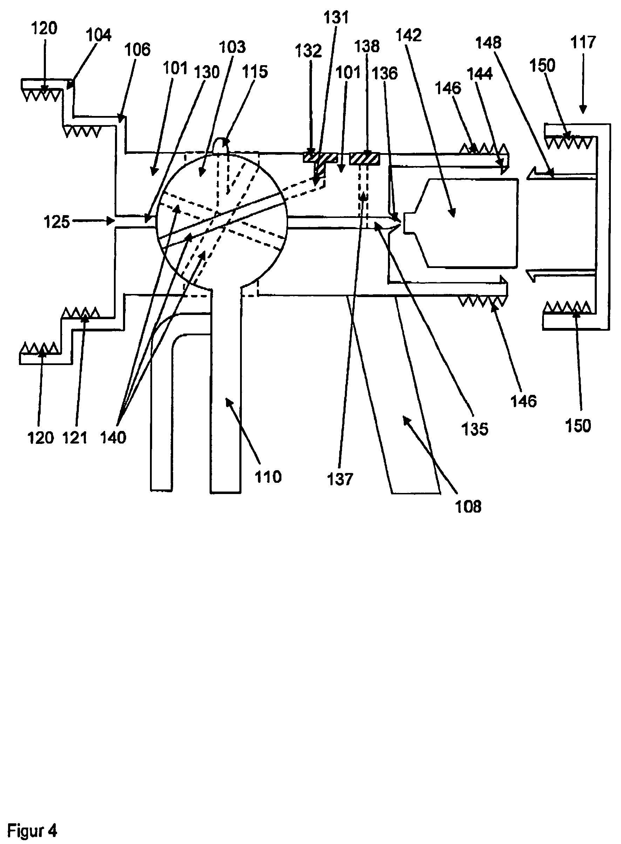

Figur 4 zeigt eine Querschnittansicht einer zweiten erfindungsgemäßen Austragsvorrichtung mit einem Grundkörper (101) an dessen Vorderseite ein erstes Kartuschenanschlussstück (104) mit großem Durchmesser für Kartuschen mit großem Durchmesser und ein zweites Kartuschenanschlussstück (106) mit kleinerem Durchmesser für Kartuschen mit kleinerem Durchmesser angeordnet sind. In einer Öffnung (nicht gezeigt) im Grundkörper (101) befindet sich ein Ventilkörper (103) mit rotationssymmetrischer Geometrie, der um seine Symmetrieachse (senkrecht zur in Figur 4 dargestellten Schnittebene) drehbar im Grundkörper (101) gelagert ist

An der Unterseite der Austragsvorrichtung ist ein Pistolengriff (108) angeordnet, mit dem die Austragsvorrichtung mit einer Hand haltbar ist. An der Unterseite des Ventilkörpers (103) ist ein Abzugsgriff (110) zum Drehen des Ventilkörpers (103) in der Öffnung des Grundkörpers (101) angeordnet, der vor dem Pistolengriff (108) platziert ist. Der Abzugsgriff (110) ist U-förmig gestaltet, so dass die Finger einer Hand in den Zwischenraum greifen können. Die Anordnung des Abzugsgriffs (110) zum Pistolengriff (108) ist derart gewählt, dass wenn der Pistolengriff (108) in einer Hand gehalten wird, der Abzugsgriff (110) mit den Fingern derselben Hand bedienbar ist. Die U-förmige Gestaltung des Abzugsgriffs (110) erlaubt die Bewegung des Ventilkörpers (103) in beide Drehrichtungen.

Auf der Oberseite des Ventilkörpers (103) ist ein Positionsanzeiger (115) angeordnet, mit dessen Hilfe sich die Position des Ventilkörpers (103) in der Austragsvorrichtung erkennen lässt. Eine Verschlusskappe (117) ist lösbar mit der Rückseite des Grundkörpers (101) zu verbinden. Fixierungsvorrichtungen (120, 121) in Form von Innengewinden sind in den Kartuschenanschlussstücken (104, 106) zur Fixierung von Kartuschen mit Außengewinden (nicht gezeigt) vorgesehen. Im vorderen Tell des Grundkörpers (101) ist ein vorderer Kanal (130) angeordnet, der in eine Öffnung (125) zu den Kartuschenanschlussstücken (104,106) und in eine breite Öffnung zum Ventilkörper (103) mündet.

Im hinteren Teil des Grundkörpers (101) sind drei hintere Kanäle (135) angeordnet, die in Öffnungen zum Ventilkörper (103) münden. Auf der der Rückseite der Austragsvorrichtung zugewandten Seite des Grundkörpers (101) münden zwei der drei hinteren Kanäle (135) in einen hohlen, beidseitig geöffneten Dorn (136) mit einem Durchlass. Der dritte hintere Kanal (135) mündet in einen ersten Ablasskanal (137), der über einen ersten Sterilfilter (138) mit der Umgebung der Austragsvorrichtung verbunden ist.

Im Ventilkörper (103) sind drei entlang der Symmetrieachse des Ventilkörpers (103) versetzte Durchgänge (140), die zusätzlich um diese Symmetrieachse gegeneinander verdreht sind, angeordnet. Die Durchgänge (140) sind derart im Ventilkörper (103) angeordnet und die Kanäle (130, 135) derart im Grundkörper (101) angeordnet, dass bei drei Stellungen des Ventilkörpers (103), die durch eine Drehung des Ventilkörpers (103) gegen den Grundkörper (101) einstellbar sind, der vordere Kanal (130) mit jeweils einem hinteren Kanal (135) über einen der Durchgänge (140) miteinander verbunden sind, wobei alle anderen hinteren Kanäle (135) durch den Ventilkörper (103) geschlossen sind. Die Breite der vorderen Öffnung zum Ventilkörper (103), die in den vorderen Kanal (130) mündet, reicht dazu aus, dass jeder der Durchgänge (140) mit dem vorderen Kanal (130) verbindbar ist

Alternativ können auch drei vordere Kanäle (130) im vorderen Teil des Grundkörpers (101) vorgesehen sein, wobei jeder vordere Kanal (130) über jeweils einen Durchgang (140) mit einem hinteren Kanal (135) verbindbar ist. Jedem der drei vorderen Kanäle (130) ist dann ein hinterer Kanal (135) und ein Durchgang (140) zugeordnet Um die Öffnungen der Kanäle (130, 135) zum Ventilkörper (103) können Dichtungen (nicht gezeigt) vorgesehen sein.

Einer der drei Durchgänge (140), die die vorderen Kanäle (130) mit den hinteren Kanälen (135) verbinden, umfasst eine Abzweigung zu einem zweiten Ablasskanal (131), der über einen zweiten Sterilfilter (132) mit der Umgebung der Austragsvorrichtung verbunden ist. Vor den Sterilfiltern (132,138) kann ein Überdruckventil angeordnet sein, dass sich ab einem gewissen Druck öffnet.

Der Dom (136) ragt in einen Hohlraum im hinteren Teil des Grundkörpers (101), der zur Aufnahme einer Gaspatrone (142) geeignet ist. Der Dorn (136) dient dazu, eine Gaspatrone (142), deren Öffnungsseite auf den Dom (136) gedrückt wird, zu öffnen. Am hinteren Ende des Hohlraums ist innenseitig eine Arretierungsvorrichtung (144) und außenseitig am Grundkörper (101) ein Befestigungsmittel (146) in Form eines Außengewindes angeordnet Die Arretierungsvorrichtung (144) dient dazu, eine ungewollte Bewegung der Verschlusskappe (117) zu verhindern. Die Arretierungsvorrichtung (144) kann dazu in Arretierungshaken (148) greifen, die an der Verschlusskappe (170) angeordnet sind. Das Befestigungsmittel (146) dient dazu, die Verschlusskappe (117) mit dem Grundkörper (101) zu verbinden, dabei die Gaspatrone (142) auf den Dom (136) zu drücken und so zu öffnen. Dazu ist an der Verschlusskappe (117) ein Befestigungsmittel (150) in Form eines Innengewindes vorgesehen.

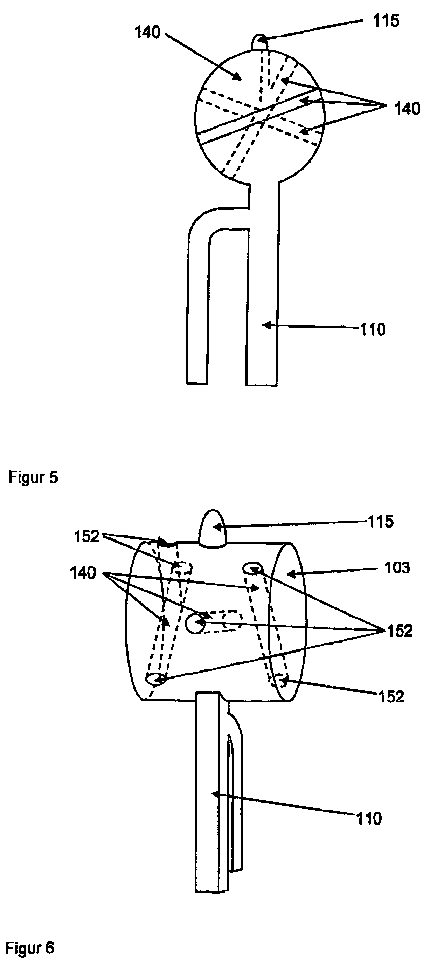

Figur 5 zeigt den Ventilkörper (103) mit Abzugsgriff (110) und Positionsanzeiger (115) nach Figur 4 in Querschnittansicht. Im Inneren des Ventilkörpers (103) befinden sich drei Durchgänge (140), die sich in unterschiedlichen Winkeln zum Abzugsgriff (110) durch den Ventilkörper (103) erstrecken. Einer der drei Durchgänge (140) umfasst einen Abzweig Im Ventilkörper (103), der im eingebauten Zustand zu einem Ablass führt, über den ein Entlüften der Kartuschenanschlussstücke (104, 106) und einer eingebauten Gaspatrone (142) möglich ist. Figur 6 zeigt eine perspektivische Ansicht des Ventilkörpers (103) mit Abzugsgriff (110) und Positionsanzeiger (115) nach Figur 5. Auf dem Zylindermantel des Ventilkörpers (103) sind Öffnungen (152) zu erkennen, in die die drei Durchgänge (140) münden. Die Durchgänge (140) sind nebeneinander angeordnet und jeweils um circa 45° gegeneinander gekippt. Einer der Durchgänge (140) hat einen Abzweig und verbindet so drei Öffnungen (152).

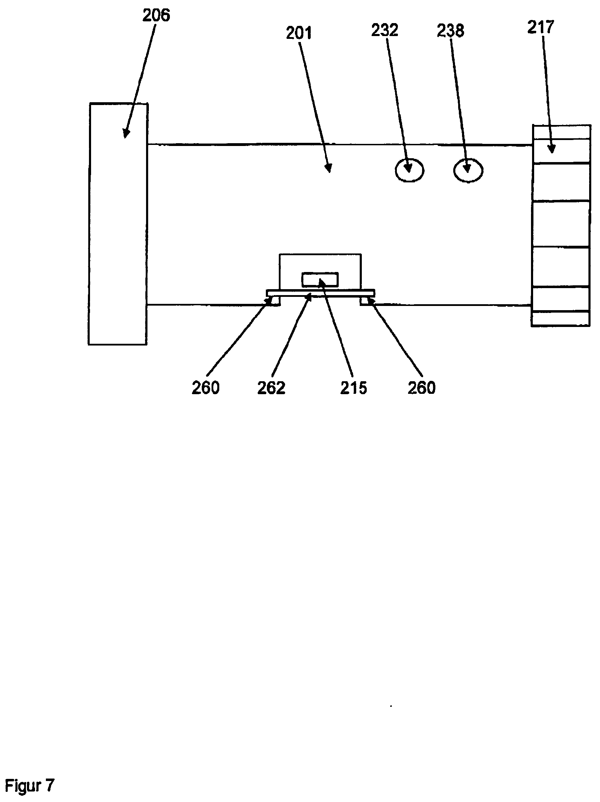

Figur 7 zeigt eine Aufsicht auf die Oberseite einer dritten erfindungsgemäßen Austragsvorrichtung umfassend einen Grundkörper (201), an dessen Vorderseite (in Figur 7 links) ein Kartuschenanschlussstück (206) angeordnet ist. Das Kartuschenanschlussstück (206) umfasst eine Zylinderwand und Befestigungsmittel (nicht gezeigt) zum Befestigen einer Kartusche (nicht gezeigt).

Auf der Rückseite der Austragsvorrichtung (in Figur 7 rechts) befindet sich eine Verschlusskapper (217), mit der ein Hohlraum im Grundkörper (201) zur Aufnahme einer Gaspatrone schließbar ist. Auf der Oberseite der Austragsvorrichtung sind zwei Sterilfilter (232, 238) im Bereich zweier Öffnungen angeordnet, die mit zwei Kanälen im Inneren des Grundkörpers (201) verbunden sind, wobei Ober die Kanäle der Hohlraum für die Gaspatrone, beziehungsweise die Gaspatrone selbst, mit dem Kartuschenanschlussstück (206) verbindbar ist.

Im Inneren des Grundkörpers (201) befindet sich ein konischer drehbarer Ventilkörper (nicht gezeigt) in einer dafür vorgesehenen, senkrecht zur Verbindungslinie Kartuschenanschlussstück (206) und Verschlusskappe (217) angeordneten konischen Bohrung Im Grundkörper (201), mit dem die verschiedenen Kanäle zwischen dem Kartuschenanschlussstück (206), dem Hohlraum für die Gaspatrone, beziehungsweise die Gaspatrone, und den Öffnungen mit den Sterilfiltern (232, 238) verbindbar und verschließbar sind. Dazu können im Ventilkörper Durchgänge angeordnet sein. Ebenso können die Durchgänge aber auch durch die Außenwand des Ventilkörpers und die Innenwand des Grundkörpers (201) in dem Bereich gebildet sein, in denen der Grundkörper (201) und der Ventilkörper miteinander in Kontakt stehen. Hierzu können Furchen in den Grenzbereichen zwischen dem Grundkörper (201) und dem Ventilkörper in deren Oberflächen vorgesehen sein. Auch eine Kombination von Furchen und Durchgängen im Inneren des Ventilkörpers kommen als Durchgänge des Ventils in Betracht.

Auf der Oberseite des Ventilkörpers befindet sich ein Positionsanzeiger (215) über den die Stellung des Ventilkörpers ablesbar ist und damit welcher der Kanäle geöffnet ist, oder ob das Ventil geschlossen ist. In der Oberseite des Grundkörpers ist eine Ausnehmung (260) senkrecht zur konischen Bohrung, die zur Aufnahme des Ventilkörpers dient, angebracht, die einen Keil (262) aufnehmen kann. Die Ausnehmung (260) ist so angeordnet, dass bei Einpressen des Keils (262) der konische Ventilkörper in die konische Bohrung gepresst wird.

Der Ventilkörper ist konisch von einer Seite zur anderen verjüngt und an der größeren Seite durch den in den Grundkörper (201) in der Ausnehmung (260) eingepressten Keil (262) fixiert. Dadurch kann ein solcher Anpressdruck des Ventilkörpers in der Bohrung erreicht werden, dass die angestrebte Dichtungsfunktion gewährleistet wird. Der Keil (262) ist selbst durch in der Kunststofftechnik übliche Schnapp- oder Rastvorrichtungen gegen unerwünschte Bewegungen in Richtung der Keilachse gesichert. Alternativ ist es auch möglich, den Ventilkörper mit mindestens einem Außengewinde zu versehen und durch ein an mindestens einer Außenseite des Grundkörpers angeordnete Mutter durch Verschraubung anzupressen und zu sichern.
Auf der Rückseite erfindungsgemäßer Austragsvorrichtungen (in den Figur 1,4 und 7 rechts) kann sich hinter dem drehbaren Ventilkörper (3,103) im Grundkörper (1, 101, 201) ein Hohlraum für eine Gaspatrone befinden. Der Grundkörper (1, 101, 201) ist zum Einsetzen oder Herausnehmen der Gaspatrone zur Rückseite der Austragsvorrichtung hin offen. Diese Öffnung kann durch eine Verschlusskappe (17, 117, 217) abdeckbar sein. Die Verschlusskappe (17, 117, 217) kann dazu die Form eines einseitig geschlossenen Hohlzylinders haben und kann mit Befestigungsmitteln am Grundkörper (1, 101, 201) befestigt werden.

Erfindungsgemäß kann es auch sein, dass ein Grundkörper (1,101,201) vorhanden ist, in dem mindestens eine Bohrung (2) angebracht ist, dass eine Seite des Grundkörpers (1, 101, 201) als Kartuschenanschlussstück (4, 104, 6, 106, 206) mit mindestens zwei in konzentrischen Kreisen angeordneten Fixierungsvorrichtungen (20, 120, 21, 121) zur Fixierung von Kartuschen angeordnet ist, die Gewinde oder Gewindeausschnitte bilden, dass eine andere Seite des Grundkörpers (1, 101, 201) als Pistolengriff (8, 108) ausgebildet ist, dass in der Bohrung (2) ein Ventilkörper (3, 103) um seine Zylinderachse drehbar angeordnet ist, dass der Ventilkörper (3, 103) ein Ventil bildet, das a) aus dem Ventilkörper (3, 103) mit mindestens zwei Durchgängen (140) b) einem Positionsanzeiger (15, 115, 215) und (c) einem Abzugsgriff (10,110) aufgebaut ist, wobei der Ventilkörper (3, 103) mit dem Positionsanzeiger (15, 115, 215) und dem Abzugsgriff (10, 110) verbunden ist, dass die Durchgänge (140) im Ventilkörper (3, 103) nicht auf einer Ebene angeordnet sind, dass zumindest zwei vordere Kanäle (130) im Grundkörper (1, 101, 201) angeordnet sind, welche zumindest zwei gasdurchlässige Durchlässe zwischen der Außenseite vom Kartuschenanschlussstück (4, 104, 6, 106, 206) und den Durchgängen (140) bilden, die vorderen Kanäle (130) münden an der Außenseite der Kartuschenanschlussstücke (4, 104, 6, 106, 206) in den Öffnungen (25, 125), die sich innerhalb der in konzentrischen Kreisen angeordneten Fixierungsvorrichtungen (20, 120, 21, 121) zur Fixierung von Kartuschen befinden, dass zumindest ein Ablasskanal (131) mit einem der Durchgänge (140) des Ventils mit einem drucksensitiven Sicherheitsventil verbindbar ist, das bei zuvor eingestelltem Gasdruck, eine Verbindung zwischen einem vorderen Kanal (130) und einem hinteren Kanal (135) und der äußeren Umgebung herstellt, dass zwischen dem Sicherheitsventil und der Umgebung ein Sterilfilter (132) angeordnet ist, dass mindestens ein Durchgang (140) des Ventilkörpers (3, 103) mit einem hinteren Kanal (135) kommunizieren kann, der mit der Gaspatrone (142) verbunden ist, dass der hintere Kanal (135) mit mindestens einem Durchgang (140) des Ventilkörpers (3, 103) und einem vorderen Kanal (130) kommunizieren kann, dass ein zweiter hinterer Kanal (135) mit einem daran angeschlossenen Ablasskanal (137) eine Verbindung zwischen mindestens einem Durchgang (140) im Ventilkörper (3,103) und der Umgebung herstellt dass zwischen dem Ablasskanal (137) und der Umgebung ein Sterilfilter (138) angeordnet ist, dass der zweite hintere Kanal (135) über mindestens einen Durchgang (140) Im Ventilkörper (3, 103) mit einem zweiten vorderen Kanal (130) gasdurchlässig verbunden werden kann.

Eine vorteilhafte Ausgestaltung der Erfindung kann zusätzlich darin bestehen, dass die Gasentnahmeöffnung der Gaspatrone (142) durch einen einsteckbaren, herausziehbaren Stift oder durch eine herausdrehbare Platte so gesichert ist, dass die Gaspatrone (142) vor der Applikation nicht durch Einwirkung von mechanischen Kräften, wie sie beim Transport auftreten können, aufgestochen wird. Daneben ist es auch möglich, den Ventilkörper (3, 103) durch einen herausziehbaren Stift oder eine Platte gegen die unbeabsichtigte Verdrehung zu schützen. Besonders vorteilhaft ist es, wenn die Sicherung der Gaspatrone (142) und des Ventilkörpers (3, 103) eine Einheit bilden und gemeinsam mit möglichst einen Handgriff vor der Applikation entfernt oder inaktiviert werden können. Die Entsicherung der Austragsvorrichtung kann zum Beispiel mit dem Öffnen der Gaspatrone (142) gekoppelt sein.

Der Grundkörper (1, 101, 201) der Austragsvorrichtung bildet bevorzugt mit dem Kartuschenanschlussstück mit zwei in Kreisen angeordneten Fixierungsvorrichtungen (20, 21, 120, 121) zur Fixierung von Kartuschen und dem Pistolengriff (8, 108) eine Einheit. Diese Teile sind als zusammenhängende Einheit bevorzugt als ein Spritzgießteil ausgebildet.

Die Anordnung von zwei Fixierungsvorrichtungen (20, 21, 120, 121) zur Fixierung von Kartuschen in zwei konzentrischen Ringen hat den Vorteil, dass mit dem inneren Ring Kartuschen mit einem entsprechenden kleinen Durchmesser, wie er bei einzelnen Kartuschen üblich ist, fixiert werden können und dass bei Verwendung von Kartuschen mit größerem Durchmesser, zum Beispiel bei Mehrkomponentenkartuschen, auch diese mit der derselben Austragsvorrichtung verbunden werden können. Der Vorteil besteht darin, dass mit einer solchen Austragsvorrichtung Kartuschen unterschiedlicher Durchmesser ausgepresst werden können. Dadurch kann die Wirtschaftlichkeit der Austragsvorrichtung verbessert werden.

Neben der Anordnung von Fixierungsvorrichtungen (20, 21, 120, 121) zur Fixierung von Kartuschen in zwei konzentrischen Ringen ist es auch im Rahmen der Erfindung, dass die Fixierungsvorrichtungen (20, 21, 120, 121) auch in nicht zueinander konzentrischen Ringen oder auch in nicht kreisförmigen Anordnungen ausgebildet sein können.

Das Ventil ist durch einen mit mindestens zwei Durchgängen durchbohrten Ventilkörper (3, 103) gebildet an den ein Positionsanzeiger (15, 115, 215) und ein Abzugsgriffstück (10, 110) angebracht sind. Das gesamte Ventil einschließlich des Positionsanzeigers (15,115,215) und des Abzugsgriffstücks (10, 110) ist bevorzugt einteilig und kann als Einzelteil durch Spritzgießen gefertigt werden. Bei Ziehen des Abzugsgriffstücks (10, 110) in Richtung Pistolengriff (8, 108) wird der Ventilkörper (3, 103) so gedreht, dass die vorderen Kanäle (130) und die hinteren Kanäle (135) durch die Durchgänge (140) gasdurchlässig verbunden sind. Bei zuvor geöffneter Gaspatrone erfolgt dann ein Gasfluss in Richtung der Kartuschenanschlussstücke (4, 104, 6, 106, 206). Das Gas strömt aus der Öffnung (25, 125) und drückt auf den Kolben der zuvor eingesetzten Kartusche. Dadurch wird der Kolben in Richtung Kartuschenkopf bewegt und presst dadurch das fließfähige Material aus der Kartusche.

Wenn das Abzugsgriffstück (10, 110) in Richtung des Kartuschenanschlussstücks (4, 104, 6, 106, 206) bewegt wird, so dreht sich der Ventilkörper (3, 103) so, dass der Durchgang (140) nicht mehr die durchgehenden vorderen und hinteren Kanäle (130,135) verbindet. Bei senkrechter Stellung des Abzugsgriffstücks (10, 110) gibt es keine Verbindung der Kanäle (130, 135) miteinander. Wenn das Abzugsgriffstück (10,110) weiter in Richtung Kartuschenanschlussstück (4, 104, 6, 106, 206) gedreht wird, verbindet ein Durchgang (140) einen durchgehenden vorderen Kanal (130) und den mit dem ersten Ablasskanal (137) verbundenen hinteren Kanal (135) miteinander. Das komprimierte Gas strömt aus dem Kartuschenanschlussstück (4, 104, 6, 106, 206) durch eine der Öffnungen (125) durch einen der durchgehenden vorderen Kanäle (130), durch einen der Durchgänge (140) und durch den ersten Ablasskanal (137) zum Sterilfilter (138). Das Gas tritt durch den Sterilfilter (138) in die Umgebung aus. Dadurch kann komprimiertes Gas aus dem Raum zwischen dem Kartuschenkolben und dem Kartuschenanschlussstück (4, 104, 6, 106, 206) über den Sterilfilter (138) in die Umgebung abgegeben werden. Die Bewegung des Kolbens wird dadurch sofort gestoppt.

Der an der Oberseite der Austragsvorrichtung herausragende Positionsanzeiger (15, 115, 215) wird automatisch bei Betätigung des Abzuggriffstücks (10, 110) mit bewegt, weil der Positionsanzeiger (15, 115, 215) auf der Oberseite des Ventilkörpers (3, 103) angeordnet ist. Das bedeutet, der Positionsanzeiger (15, 115, 215) zeigt den Verschlusszustand des Ventils an. Der Anwender erhält somit jederzeit bei Gebrauch der Austragsvorrichtung die Information über den Verschlusszustand des Ventils.

Es ist erfindungsgemäß, dass die Durchgänge (140) im Ventilkörper (3, 103) um einen Winkel von mindestens 10 ° versetzt in einer Ebene senkrecht zur Achse des Zylinders angeordnet sind.

Weiterhin kann erfindungsgemäß vorgesehen sein, dass im Grundkörper (1, 101, 201) vordere Kanäle (130) und hintere Kanäle (135) angeordnet sind, wobei der erste vordere Kanal (130) die Außenseite des Kartuschenanschlussstücks (4, 104, 6, 106, 206) mit einem ersten Durchgang (140) im Ventilkörper (3, 103) verbindet, wobei der erste hintere Kanal (135) den ersten Durchgang (140) mit einem Sterilfilter (138) verbindet, der mit der Umgebungsatmosphäre verbunden ist und das der Ventilkörper (3, 103) einen zweiten Durchgang (140) umfasst, welche einen zweiten vorderen Kanal (130) und einen zweiten hinteren Kanal (135) so miteinander verbindet, dass eine Gasdurchströmung gewährleistet ist.

Das Abzugsgriffstück (10, 110) ist bevorzugt U-förmig ausgebildet. Dadurch ist es möglich, mit einer einfachen Bewegung mit der Hand das Griffstück in Richtung Kartuschenanschlussstück zu drücken und damit den Gasfluss und damit den Fluss des pastösen Materials zu stoppen. Bei weiterer Bewegung des Abzugsgriffstücks (10,110) in Richtung Kartuschenanschlussstück (4, 104, 6, 106, 206) kann das komprimierte Gas über eine erste Öffnung (25,125) durch den ersten vorderen Kanal (130), durch den ersten Durchgang (140), durch den ersten hinteren Kanal (135), durch den Ablasskanal (137) und den Sterilfilter (138) in die Umgebung abgelassen werden.

Die Sterilfilter (132, 232, 138, 238) können aus medizinischem Papier, gasdurchlässigen Kunststoffmembranen, gasdurchlässigen Kunststoffporenscheiben, gasdurchlässigen Metallporenscheiben, gasdurchlässigen Glasporenscheiben oder aus gasdurchlässigen Kunststoffvliesen gefertigt sein.

Es kann auch erfindungsgemäß vorgesehen sein, dass die Bohrung (2) im Grundkörper (1, 101, 201) bevorzugt konisch von einer Außenseite zur anderen Außenseite verjüngt ist und dass im Grundkörper (1, 101, 201) auf der Seite der größeren Öffnung der Bohrung (2) eine Ausnehmung (260) senkrecht zur Bohrung (2) vorgesehen ist, die einen Keil (262) aufnehmen kann. Die Ausnehmung (260) ist so angeordnet, dass bei Einpressen des Keils (262) der Ventilkörper (3, 103) in die Bohrung (2) gepresst wird.

Spezielle Ausgestaltungen der vorliegenden Erfindung können auch dadurch gekennzeichnet sein, dass der Ventilkörper (3, 103) bevorzugt konisch von einer Seite zur anderen Seite verjüngt ist und dass der Ventilkörper (3, 103) an der größeren Seite durch einen in den Grundkör per (1, 101, 201) in die Ausnehmung (260) eingepressten Keil (262) fixiert ist Dadurch kann ein solcher Anpressdruck des Ventilkörpers (3, 103) in der Bohrung (2) erreicht werden, dass die angestrebte Dichtungsfunktion gewährleistet wird. Der Keil (262) kann selbst durch in der Kunststofftechnik übliche Schnapp- oder Rastvorrichtungen gegen unerwünschte Bewegungen in Richtung der Keilachse gesichert sein. Alternativ ist es auch möglich, den Ventilkörper (3, 103) mit mindestens einem Außengewinde an den Zylinderende zu versehen und durch ein an mindestens einer Außenseite des Grundkörpers (1, 101, 201) angeordnete Mutter durch Verschraubung anzupressen und zu sichern.

Erfindungsgemäße Austragsvorrichtungen sind bevorzugt im Wesentlichen aus medizinisch akzeptablen Kunstoffen gefertigt.

Erfindungsgemäße Austragsvorrichtungen sind besonders bevorzugt zum Auspressen von fließfähigen Massen aus Kartuschen geeignet, wobei die fließfähigen Massen direkt in den Kartuschen gelagert sind, und zum Auspressen von fließfähigen Massen, die in Beuteln gelagert sind, die sich wiederum in Kartuschen befinden, geeignet

Erfindungsgemäße Austragsvorrichtungen können zum Austragen von Knochenzement, von Dentalmaterialien, von Klebstoffen und Dichtungsmitteln aus Kartuschen verwendet werden. Die erfindungsgemäße Austragsvorrichtung kann zum einmaligen und mehrmaligen Gebrauch, bestimmt sein. Aufgrund der geringen Herstellungskosten ist der einmalige Gebrauch besonders geeignet

Die in der voranstehenden Beschreibung, sowie den Ansprüchen, Figuren und Ausführungsbeispielen offenbarten Merkmale der Erfindung können sowohl einzeln, als auch in jeder beliebigen Kombination für die Verwirklichung der Erfindung in ihren verschiedenen Ausführungeformen wesentlich sein.

Insbesondere ist es für die Ausführung des drehbaren Ventilkörpers (3,103) nicht notwendig, dass mehr als ein Kartuschenanschlussstück (4, 104, 6, 106, 208) vorgesehen ist.

### Bezugszeichenliste

- 1, 101, 201: Grundkörper
- 2: Öffnung
- 3, 103: Ventilkörper
- 4, 104: Kartuschenanschlussstück
- 6, 106, 206: Kartuschenanschlussstück
- 8, 108: Pistolengriff
- 10, 110: Abzugsgriff
- 15, 115, 215: Positionsanzeiger
- 17, 117, 217: Verschlusskappe
- 20, 120: Fixierungsvorrichtung
- 21, 121: Fixierungsvorrichtung
- 25, 125: Öffnung im Kartuschenenschlussstück
- 26: Rastvorrichtung
- 130: vorderer Kanal
- 131, 137: Ablasskanal
- 132, 232: Sterilfilter
- 135: hinterer Kanal
- 136: hohler Dom
- 138, 238: Sterilfilter
- 140: Durchgang
- 142: Gaspatrone
- 144: Arretierungsvorrichtung
- 146: Befestigungsmittel
- 148: Arretierungshaken
- 150: Befestigungsmittel
- 152: Öffnungen im Ventilkörper
- 260: Ausnehmung
- 262: Keil

## Patentansprüche

1. Austragsvorrichtung für Kartuschen umfassend einen Abzugsgriff (10, 110) zum Bedienen eines Ventils der Austragsvorrichtung, einen Anschluss (136) zum Einspeisen eines unter Druck stehenden Gases, ein Kartuschenanschlussstück (6, 106, 206) zum Anschließen einer Kartusche und einen Grundkörper (1, 101, 201), in dem wenigstens ein Durchgang (140) verläuft, durch den das unter Druck stehende Gas zum Kartuschenanschlussstück (6, 106, 206) leitbar ist, wobei das Kartuschenanschlussstück (6, 106, 206) eine Öffnung (25, 125) zu dem Durchgang (140) umfasst, so dass bei Beaufschlagung eines Bodens einer im Kartuschenanschlussstück (6, 106, 206) angeordneten Kartusche mit dem unter Druck stehenden Gas, der Inhalt aus der Kartusche austreibbar ist, und wobei der Durchgang (140) durch das bedienbare Ventil schließbar und zu öffnen ist, **dadurch gekennzeichnet, dass**
das Ventil ein Ventilkörper (3, 103) ist, der drehbar im Grundkörper (1, 101, 201) angeordnet ist, wobei eine Bewegung des Abzugsgriffs (10, 110) zu einer Drehung des Ventilkörpers (3, 103) im Grundkörper (1, 101, 201) führt und der Durchgang (140) im oder am Ventilkörper (3, 103) angeordnet ist, so dass in einer ersten Position des Ventilkörpers (3, 103) der Durchgang (140) geschlossen ist und in einer zweiten Position des Ventilkörpers (3, 103) der Durchgang (140) im oder am Ventilkörper (3, 103) das unter Druck stehende Gas zu dem Kartuschenanschlussstück (6, 106, 206) leitet, und
ein zweiter Durchgang (140) im oder am Ventilkörper (3, 103) angeordnet ist, der in einer dritten Position des Ventilkörpers (3, 103) das Kartuschenanschlussstück (6, 106, 206) mit einem ersten Ablass verbindet, so dass ein Überdruck am Kartuschenanschlusstück (6, 106, 206) in der dritten Position des Ventilkörpers (3, 103) durch den ersten Ablass entweichen kann.

2. Austragsvorrichtung nach Anspruch 1, **dadurch gekennzeichnet, dass** zwischen zumindest einem Durchgang (140) und dem Kartuschenanschlussstück (6, 106, 206) zumindest ein vorderer Kanal (130) im Grundkörper (1, 101, 201) angeordnet ist und/oder zwischen zumindest einem Durchgang (140) und dem Anschluss (136) zum Einspeisen des unter Druck stehenden Gases zumindest ein hinterer Kanal (135) angeordnet ist.

3. Austragsvorrichtung nach Anspruch 2, **dadurch gekennzeichnet, dass**
ein erster Ablasskanal (137) im Grundkörper (1, 101, 201) derart angeordnet ist, dass er in der dritten Position des Ventilkörpers (3, 103) den zweiten Durchgang (140) mit dem ersten Ablass verbindet.

4. Austragsvorrichtung nach einem der vorangehenden Ansprüche, **dadurch gekennzeichnet, dass**
ein dritter Durchgang (140) im oder am Ventilkörper (3, 103) angeordnet ist, der einen Abzweig umfasst und der in einer vierten Position des Ventilkörpers (3, 103) das Kartuschenanschlussstück (6, 106, 206) und das unter Druck stehende Gas mit einem zweiten Ablass verbindet, so dass ein Überdruck in der Austragsvorrichtung in der vierten Position des Ventilkörpers (3, 103) durch den zweiten Ablass entweichen kann.

5. Austragsvorrichtung nach Anspruch 4, **dadurch gekennzeichnet, dass**
zwischen dem dritten Durchgang (140) und dem Anschluss (136) zum Einspeisen des unter Druck stehenden Gases ein zweiter hinterer Kanal (135) im Grundkörper (1, 101, 201) angeordnet ist und/oder ein zweiter Ablasskanal (131) im Grundkörper (1, 101, 201) derart angeordnet ist, dass er in der vierten Position des Ventilkörpers (3, 103) den Abzweig des dritten Durchgangs (140) mit dem zweiten Ablass verbindet.

6. Austragsvorrichtung nach einem der Ansprüche 2 bis 5, **dadurch gekennzeichnet, dass** an zumindest einem der Ablässe ein Sterilfilter (132, 138) angeordnet ist und/oder in zumindest einem der Ablässe ein Überdruckventil angeordnet ist.

7. Austragsvorrichtung nach einem der vorangehenden Ansprüche, **dadurch gekennzeichnet, dass**
der Abzugsgriff (10, 110) fest an der Unterseite des drehbaren Ventilkörpers (3, 103) angeordnet ist, so dass mit dem Abzugsgriff (10, 110) der Ventilkörper (3, 103) im Grundkörper (1, 101, 201) drehbar ist.

8. Austragsvorrichtung nach einem der vorangehenden Ansprüche, **dadurch gekennzeichnet, dass**
am drehbaren Ventilkörper (3, 103) ein Positionsanzeiger (15, 115, 215), insbesondere an der Oberseite, angeordnet ist, an dem die Position des Ventilkörpers (3, 103) im Grundkörper (1, 101, 201) ablesbar ist.

9. Austragsvorrichtung nach einem der vorangehenden Ansprüche, **dadurch gekennzeichnet, dass**
an der Unterseite des Grundkörpers (1, 101, 201) ein Griff (8, 108) zum Halten der Austragsvorrichtung angeordnet ist.

10. Austragsvorrichtung nach einem der vorangehenden Ansprüche, **dadurch gekennzeichnet, dass**
der Ventilkörper (3, 103) ein konisch geformter, zumindest bereichsweise rotationssymmetrischer Körper ist, der in einer zur Form des Ventilkörpers (3, 103) passenden konischen Öffnung (2) im Grundkörper (1, 101, 201) drehbar gelagert ist, wobei die Außenwände des Ventilkörpers (3, 103) bis auf die Öffnungen der Durchgänge (140) dicht mit den Wänden der konischen Öffnung (2) im Grundkörper (1, 101, 201) in jeder Position des Ventilkörpers (3, 103) abschließen, so dass die Verbindungen der Durchgänge (140) zu zumindest einem Kanal (130, 131, 135, 137) im Grundkörper (1, 101, 201) dicht sind.

11. Austragsvorrichtung nach Anspruch 10, **dadurch gekennzeichnet, dass**
im Grundkörper (1, 101, 201) eine Ausnehmung (260) für einen Keil (262) vorgesehen ist, wobei ein in die Ausnehmung (260) eingeschobener Keil (262) den Ventilkörper (3, 103) in die konische Öffnung (2) im Grundkörper (1, 101, 201) presst, so dass der Ventilkörper (3, 103) einen gasdichten Presssitz in der konischen Öffnung (2) erzeugt.

12. Austragsvorrichtung nach einem der vorangehenden Ansprüche, **dadurch gekennzeichnet, dass**
an der dem Kartuschenanschlussstück (6, 106, 206) abgewandten Seite des Grundkörpers (1, 101, 201) ein Hohlraum für eine Gaspatrone vorgesehen ist, wobei der Anschluss (136) in dem Hohlraum angeordnet ist und einen Dorn zum Öffnen einer Gaspatrone umfasst, und am Grundkörper (1, 101, 201) Befestigungsmittel (146) zum Befestigen einer Verschlusskappe (17, 117, 217) mit Befestigungsmitteln (150) angeordnet sind, wobei die Gaspatrone (142) durch das Befestigen der Verschlusskappe (17, 117, 217) zu öffnen ist, wobei vorzugsweise die Verschlusskappe (17, 117, 217) zusätzlich ein Arretierungsmittel (148) umfasst, das in eine Arretierungsvorrichtung (144) im Hohlraum greift und wobei besonders bevorzugt die Arretierungsvorrichtung (144) eine eingesetzte Gaspatrone (142) geeigneter Größe im Hohlraum positioniert.

13. Austragsvorrichtung nach einem der Ansprüche 2 bis 12, **dadurch gekennzeichnet, dass** die Durchgänge (140) im Wesentlichen gerade durch das Innere des Ventilkörpers (3, 103) verlaufen und die Durchgänge (140) um einen Winkel von 10° bis 80° um die Drehachse des Ventilkörpers (3, 103) gegeneinander versetzt sind.

14. Austragsvorrichtung nach einem der vorangehenden Ansprüche, **dadurch gekennzeichnet, dass**
am vorderen Rand des ersten Kartuschenanschlussstücks (6, 106, 206) ein zweites Kartuschenanschlussstück (4, 104) mit einem größeren Querschnitt und einer zweiten Öffnung angeordnet ist, wobei das zweite Kartuschenanschlussstück (4, 104) zum Anschließen einer größeren Kartusche geeignet ist, und wobei die zweite Öffnung des zweiten Kartuschenanschlussstücks (4, 104, 204) so groß ist, dass eine Kartusche durch die zweite Öffnung hindurch am ersten Kartuschenanschlussstück (6, 106, 206) anschließbar ist.

15. Austragsvorrichtung nach Anspruch 14, **dadurch gekennzeichnet, dass**
die Kartuschenanschlussstücke (4, 104, 6, 106, 206) zylindrische Hohlkörper mit einer geschlossenen Seite sind, wobei die geschlossene Seite des zweiten Kartuschenanschlussstücks (4, 104) die zweite Öffnung umfasst und die geschlossene Seite des ersten Kartuschenanschlussstücks (6, 106, 206) die erste Öffnung (25, 125) umfasst.

16. Austragsvorrichtung nach Anspruch 14 oder 15, **dadurch gekennzeichnet, dass**
auf der Innenseite der Kartuschenanschlussstücke (4, 104, 6, 106, 206) Fixierungsvorrichtungen (20, 120, 21, 121) zum Fixieren von Kartuschen angeordnet sind.

17. Austragsvorrichtung nach Anspruch 16, **dadurch gekennzeichnet, dass**
die Fixierungsvorrichtungen (20, 120, 21, 121) in zwei konzentrischen Kreisen an den Kartuschenanschlussstücken (4, 104, 6, 106, 206) angeordnet sind.

18. Austragsvorrichtung nach Anspruch 16 oder 17, **dadurch gekennzeichnet, dass** die Fixierungsvorrichtungen (20, 120, 21, 121) Gewinde, insbesondere Innengewinde, sind.

19. Austragsvorrichtung nach Anspruch 16 oder 17, **dadurch gekennzeichnet, dass**
die Fixierungsvorrichtungen (20, 120, 21, 121) Rastvorrichtungen (26), insbesondere in Form von in eine Richtung deformierbarer Zapfen umfassen.

## Claims

1. Dispensing device for cartridges comprising a trigger grip (10, 110) for operating a valve of the dispensing device, a connector (136) for supplying a pressurised gas, a cartridge connector (6, 106, 206) for connecting a cartridge, and a base body (1, 101, 201), in which at least one passage (140) extends through which the pressurised gas can be guided to the cartridge connector (6, 106, 206), whereby the cartridge connector (6, 106, 206) comprises an opening (25, 125) towards the passage (140) such that the content can be expelled from the cartridge upon application of the pressurised gas to the floor of a cartridge that is arranged in the cartridge connector (6, 106, 206), and whereby the passage (140) can be closed and opened through the operable valve, **characterised in that**
the valve is a valve body (3, 103) that is arranged in the base body (1, 101, 201) such as to be rotatable, whereby a motion of the trigger grip (10, 110) leads to a rotation of the valve body (3, 103) in the base body (1, 101, 201) and the passage (140) is arranged in or on the valve body such that the passage (140) is closed in a first position of the valve body (3, 103) and the passage (140) in or on the valve body (3, 103) guides the pressurised gas to the cartridge connector (6, 106, 206) in a second position of the valve body (3, 103), and
a second passage (140) is arranged in or on the valve body (3, 103), which connects the cartridge connector (6, 106, 206) to a first drain in a third position of the valve body (3, 103) such that any over-pressure on the cartridge connector (6, 106, 206) can escape through the first drain in the third position of the valve body (3, 103).

2. Dispensing device according to claim 1, **characterised in that** at least one front channel (130) is arranged in the base body (1, 101, 201) between at least one passage (140) and the cartridge connector (6, 106, 206) and/or at least one rear channel (135) is arranged between at least one passage (140) and the connector (136) for supplying the pressurised gas.

3. Dispensing device according to claim 2, **characterised in that** a first drain channel (137) is arranged in the base body (1, 101, 201) in a manner such that it connects the second passage (140) to the first drain in the third position of the valve body (3, 103).

4. Dispensing device according to any one of the preceding claims, **characterised in that**,
a third passage (140) is arranged in or on the valve body (3, 103), which third passage comprises a branch and connects the cartridge connector (6, 106, 206) and the pressurised gas to a second drain in the fourth position of the valve body (3, 103) such that an over-pressure in the dispensing device can escape through the second drain in the fourth position of the valve body (3, 103).

5. Dispensing device according to claim 4, **characterised in that**
a second rear channel (135) is arranged in the base body (1, 101, 201) between the third passage (140) and the connector (136) for supplying the pressurised gas and/or a second drain channel (131) is arranged in the base body (1, 101, 201) in a manner such that it connects the branch of the third passage (140) to the second drain in the fourth position of the valve body (3, 103).

6. Dispensing device according to any one of the claims 2 to 5, **characterised in that**
a sterile filter (132, 138) is arranged on at least one of the drains and/or a pressure relief valve is arranged in at least one of the drains.

7. Dispensing device according to any one of the preceding claims, **characterised in that**,
the trigger grip (10, 110) is connected in fixed manner to the underside of the rotatable valve body (3, 103) such that the valve body (3, 103) can be rotated in the base body (1, 101, 201) by means of the trigger grip (10, 110).

8. Dispensing device according to any one of the preceding claims, **characterised in that**,
a position indicator (15, 115, 215) is arranged on the rotatable valve body (3, 103), in particular on the top side, on which the position of the valve body (3, 103) in the base body (1, 101, 201) can be detected.

9. Dispensing device according to any one of the preceding claims, **characterised in that**,
a handle (8, 108) allowing the dispensing device to be held is arranged on the underside of the base body (1, 101, 201).

10. Dispensing device according to any one of the preceding claims, **characterised in that**,
the valve body (3, 103) is a conically-shaped body that is rotationallysymmetrical at least over parts of it, and is mounted in rotatable manner in a conical opening (2) in the base body (1, 101, 201) that matches the shape of the valve body (3, 103), whereby the external walls of the valve body (3, 103), except for the openings of the passages (140), end in a sealed manner against the walls of the conical opening (2) in the base body (1, 101, 201) in each position of the valve body (3, 103) such that the connections of the passages (140) to at least one channel (130, 131, 135, 137) in the base body (1, 101, 201) are sealed.

11. Dispensing device according to claim 10, **characterised in that**
a recess (260) for a wedge (262) is provided in the base body (1, 101, 201), whereby a wedge (262) that is inserted into the recess (260) presses the valve body (3, 103) into the conical opening (2) in the base body (1, 101, 201) such that the valve body (3, 103) generates a gas-tight press-fit in the conical opening (2).

12. Dispensing device according to any one of the preceding claims, **characterised in that**,
a hollow space for a gas cartridge is provided on the side of the base body (1, 101, 201) facing away from the cartridge connector (6, 106, 206), whereby the connector (136) is arranged in the hollow space and comprises a mandrel for opening a gas cartridge, and fastening means (146) for fastening a closure cap (17, 117, 217) with fastening means (150) are arranged on the base body (1, 101, 201), whereby the gas cartridge (142) can be opened by the closure cap (17, 117, 217) being fastened, whereby the closure cap (17, 117, 217) preferably comprises an additional locking means (148) that engages a locking device (144) in the hollow space, and whereby it is particularly preferred for the locking device (144) to position an inserted gas cartridge (142) of appropriate size inside the hollow space.

13. Dispensing device according to any one of the claims 2 to 12, **characterised in that**
the passages (140) extend essentially straight through the inside of the valve body (3, 103) and the passages (140) are offset with respect to each other by an angle of 10° to 80° about the rotation axis of the valve body (3, 103).

14. Dispensing device according to any one of the preceding claims, **characterised in that**,
a second cartridge connector (4, 104) having a larger cross-section and a second opening is arranged at the front edge of the first cartridge connector (6, 106, 206), whereby the second cartridge connector (4, 104) is suitable for connecting a larger cartridge, and whereby the second opening of the second cartridge connector (4, 104, 204) is sufficiently large to allow a cartridge to be connected to the first cartridge connector (6, 106, 206) proceeding through the second opening.

15. Dispensing device according to claim 14, **characterised in that**
the cartridge connectors (4, 104, 6, 106, 206) are cylindrical hollow bodies having one closed side, whereby the closed side of the second cartridge connector (4, 104) comprises the second opening and the closed side of the first cartridge connector (6, 106, 206) comprises the first opening (25, 125).

16. Dispensing device according to claim 14 or 15, **characterised in that**
fixation devices (20, 120, 21, 121) for fixing cartridges in place are arranged on the inside of the cartridge connectors (4, 104, 6, 106, 206).

17. Dispensing device according to claim 16, **characterised in that**
the fixation devices (20, 120, 21, 121) are arranged in two concentric circles on the cartridge connectors (4, 104, 6, 106, 206).

18. Dispensing device according to claim 16 or 17, **characterised in that**
the fixation devices (20, 120, 21, 121) are threads, in particular internal threads.

19. Dispensing device according to claim 16 or 17, **characterised in that**
the fixation devices (20, 120, 21, 121) comprise snap-in locking devices (26), in particular in the form of pegs that can be deformed in one direction.

## Revendications

1. Dispositif de distribution pour cartouches comprenant une poignée de détente (10,110) pour utiliser un clapet du dispositif de distribution, un raccord (136) pour fournir un gaz sous pression, une pièce de raccord de cartouche (6,106, 206) pour raccorder une cartouche et un corps de base (1,101, 201), dans lequel au moins un passage (140) est formé, à travers lequel le gaz sous pression peut être conduit en direction de la pièce de raccord de cartouche (6,106, 206), dans lequel la pièce de raccord de cartouche (6, 106, 206) comprend une ouverture (25,125) vers le passage (140), de sorte que lorsque le gaz sous pression est fourni à un fond d'une cartouche disposée dans la pièce de raccord de cartouche (6,106, 206), le contenu de la cartouche peut être sorti, et dans lequel le passage (140) peut être fermé et est à ouvrir par le clapet utilisable, **caractérisé en ce que**
le clapet est un corps de clapet (3, 103) qui est disposé rotatif dans le corps de base (1, 101, 201), dans lequel un mouvement de la poignée de détente (10, 110) conduit à une rotation du corps de clapet (3, 103) dans le corps de base (1, 101, 201) et le passage (140) est ainsi disposé dans ou sur le corps de clapet (3, 103), que dans une première position du corps de clapet (3, 103), le passage (140) est fermé et dans une deuxième position du corps de clapet, le passage (140) dans ou sur le corps de clapet (3, 103) conduit le gaz sous pression vers la pièce de raccord de cartouche (6, 106, 206), et
qu'un deuxième passage (140) est disposé dans ou sur le corps de clapet (3,103), qui relie la pièce de raccord de cartouche (6,106, 206) à une première purge dans une troisième position du corps de clapet (3, 103), de sorte qu'une surpression sur la pièce de raccord de cartouche (6,106, 206) dans la troisième position de corps de clapet (3, 103) peut s'échapper par la première purge.

2. Dispositif de distribution selon la revendication 1, **caractérisé en ce qu'**entre au moins un passage (140) et la pièce de raccord de cartouche (6, 106, 206), au moins un canal avant (130) est disposé dans le corps de base (1,101, 201) et/ou qu'entre au moins un passage (140) et le raccord (136) pour fournir le gaz sous pression, au moins un canal arrière (135) est disposé.

3. Dispositif de distribution selon la revendication 2, **caractérisé en ce**
**qu'**un premier canal de purge (137) est ainsi disposé dans le corps de base (1,101, 201), que dans la troisième position du corps de clapet (3, 103), il relie le deuxième passage (140) à la première purge.

4. Dispositif de distribution selon l'une des revendications précédentes, **caractérisé en ce que**
qu'un troisième passage (140) est disposé dans ou sur le corps de clapet (3, 103), qui comprend une dérivation et qui, dans une quatrième position du corps de clapet (3, 103), relie la pièce de raccord de cartouche (6,106, 206) et le gaz sous pression à une deuxième purge, de sorte qu'une surpression dans le dispositif de distribution dans la quatrième position du corps de clapet (3, 103) peut s'échapper par la deuxième purge.

5. Dispositif de distribution selon la revendication 4, **caractérisé en ce**
**qu'**un deuxième canal arrière (135) dans le corps de base (1,101, 201) est disposé entre le troisième passage (140) et le raccord (136) pour fournir le gaz sous pression, et/ou qu'un deuxième canal de purge (131) est ainsi disposé dans le corps de base (1, 101, 201), que dans la quatrième position du corps de clapet (3,103), il relie la dérivation du troisième passage (140) à la deuxième purge.

6. Dispositif de distribution selon l'une des revendications 2 à 5, **caractérisé en ce**
**que** sur au moins une des purges, un filtre stérile (132, 138) est disposé et/ou qu'une soupape de surpression est disposée dans au moins une des purges.

7. Dispositif de distribution selon l'une des revendications précédentes, **caractérisé en ce que**
la poignée de détente (10,110) est disposée fixement sur la face inférieure du corps de clapet (3, 103) rotatif, de sorte qu'avec la poignée de détente (10, 110), le corps de clapet (3, 103) peut tourner dans le corps de base (1, 101, 201)

8. Dispositif de distribution selon l'une des revendications précédentes, **caractérisé en ce que**
qu'un indicateur de position (15, 115, 215) est disposé sur le corps de clapet (3, 103) rotatif, en particulier sur la face supérieure, sur lequel la position du corps de clapet (3, 103) dans le corps de base (1, 101, 201) peut être lue.

9. Dispositif de distribution selon l'une des revendications précédentes, **caractérisé en ce que**
que sur la face inférieure du corps de base (1, 101, 201), il est prévu une poignée (8, 108) pour saisir le dispositif de distribution.

10. Dispositif de distribution selon l'une des revendications précédentes, **caractérisé en ce que**
le corps de clapet (3, 103) est un corps conique, au moins symétrique en rotation par tronçons, qui est disposé rotatif dans une ouverture conique (2) dans le corps de base (1,101, 201) adaptée à la forme du corps de clapet (3, 103), dans lequel les parois extérieures du corps de clapet (3, 103), à l'exception des ouvertures des passages (140) se terminent de façon étanche avec les parois de l'ouverture conique (2) dans le corps de base (1, 101, 201) dans chaque position du corps de soupape (3,103), de sorte que les liaisons des passages (140) vers au moins un canal (130, 131, 135, 137) dans le corps de base (1,101, 201) sont étanches.

11. Dispositif de distribution selon la revendication 10, **caractérisé en ce**
**qu'**un évidement (260) pour un coin (262) est prévu dans le corps de base (1, 101, 201), dans lequel un coin (262) poussé dans l'évidement (260) appuie le corps de clapet (3, 103) dans l'ouverture conique (2) dans le corps de base (1, 101, 201), de sorte que le corps de clapet produit un ajustage serré étanche aux gaz dans l'ouverture conique (2).

12. Dispositif de distribution selon l'une des revendications précédentes, **caractérisé en ce que**
que sur le côté du corps de base (1,101, 201) détourné de la pièce de raccord de cartouche (6, 106, 206), il est prévu une cavité pour une cartouche de gaz, dans lequel le raccord (136) est disposé dans la cavité et comprend un poinçon pour ouvrir une cartouche de gaz, et sur le corps de base (1,101, 201), des moyens de fixation (146) sont prévus pour fixer un bouchon de fermeture (17,117, 217) avec des moyens de fixation (150), dans lequel la cartouche de gaz (142) est à ouvrir parla fixation du bouchon de fermeture (17,117,217), dans lequel de préférence, le bouchon de fermeture (17,117, 217) comprend en outre un moyen de blocage (148) qui se met en prise dans un dispositif de blocage (144) dans la cavité et dans lequel, de manière particulièrement préférée, le dispositif de blocage (144) positionne une cartouche de gaz (142) insérée, de taille appropriée, dans la cavité.

13. Dispositif de distribution selon l'une des revendications 2 à 12, **caractérisé en ce que**
les passages (140) sont essentiellement droits à travers l'intérieur du corps de clapet (3, 103) et les passages (140) sont en quinconce d'un angle de 10° à 80° sur l'axe de rotation du corps de clapet (3, 103).

14. Dispositif de distribution selon l'une des revendications précédentes, **caractérisé en ce que**
que sur le bord avant de la première pièce de raccord de cartouche (6,106, 206), il est prévu une deuxième pièce de raccord de cartouche (4, 104) avec une plus grosse section et une deuxième ouverture, dans lequel la deuxième pièce de raccord de cartouche (4,104) est appropriée pour raccorder une plus grande cartouche, et dans lequel la deuxième ouverture de la deuxième pièce de raccord de cartouche (4,104, 204) est si grande qu'une cartouche peut être raccordée sur la première pièce de raccord de cartouche (6,106, 206) par la deuxième ouverture.

15. Dispositif de distribution selon la revendication 14, **caractérisé en ce**
**que** les pièces de raccord de cartouche (4, 104, 6, 106, 206) sont des corps creux cylindriques avec un côté fermé, dans lequel le côté fermé de la deuxième pièce de raccord de cartouche (4, 104) comprend la deuxième ouverture et le côté fermé de la première pièce de raccord de cartouche (6, 106, 206) comprend la première ouverture (25,125).

16. Dispositif de distribution selon la revendication 14 ou 15, **caractérisé en ce que** des dispositifs de fixation (20, 120, 21, 121) pour fixer des cartouches sont disposés sur la face intérieure de la pièce de raccord de cartouche (4,104, 6, 106,206).

17. Dispositif de distribution selon la revendication 16, **caractérisé en ce**
**que** les dispositifs de fixation (20,120, 21, 121) sont disposés en deux cercles concentriques sur les pièces de raccord de cartouche (4, 104, 6, 106, 206).

18. Dispositif de distribution selon la revendication 16 ou 17, **caractérisé en ce que** les dispositifs de fixation (20, 120, 21, 121) sont des filetages, en particulier des filetages femelle.

19. Dispositif de distribution selon la revendication 16 ou 17, **caractérisé en ce que** les dispositifs de fixation (20, 120, 21, 121) comprennent des dispositifs d'encliquetage (26), en particulier sous forme de broches déformables dans un sens.
